# EUROPEAN PATENT SPECIFICATION

(11) **EP 4 199 910 B1**
(45) Date of publication and mention of the grant of the patent: **04.12.2024**
(21) Application number: 21763354.4
(22) Date of filing: 20.08.2021
(51) Int. Cl.: A61K 9/19, F26B 5/06

(54) **PROCEDURE OF DRYING A SUBSTRATE USING A LYOPHILISATE RETAINER**
VERFAHREN ZUM TROCKNEN EINES SUBSTRATS MIT EINEM LYOPHILISATHALTER
PROCÉDÉDE SÉCHAGE D'UN SUBSTRAT AVEC UN DISPOSITIF DE RETENUE DE LYOPHILISAT

(30) Priority: 20.08.2020 EP 20191891
(43) Date of publication of application: 28.06.2023
(73) Proprietor: F. Hoffmann-La Roche AG, 4070 Basel (CH)
(72) Inventor: KULLMANN, Daniel, 8050 Zuerich (CH); LEMA MARTINEZ, Carmen, 4070 Basel (CH); SCHWACH, Gregoire, 4070 Basel (CH); LUEMKEMANN, Joerg, 4070 Basel (CH); ZUMSTEIN, Thomas, 4070 Basel (CH); HOFER, Pascal, Markus, 4070 Basel (CH); LUZIO, Jonas, 4070 Basel (CH)
(74) Representative: Latscha Schöllhorn Partner AG
(86) International application number: PCT/EP2021/073167
(87) International publication number: WO 2022/038273

(56) References cited:
- CN-A- 110 551 998
- DE-A1- 102015 216 725
- US-A1- 2008 003 489
- US-A1- 2017 021 416

## Description

### Technical Field

The present invention relates to a procedure of drying a substrate using such a lyophilisate retainer. Retainers of that kind can be used to efficiently freeze dry the substrate and to handle the lyophilisate generated thereby.

### Background Art

In many chemical, pharmaceutical, nutritional and other applications substances are provided in a dry form. Thereby, in order to achieve a long shelf life, it is often aimed to have the substances as dry as possible. In this context, it is known to lyophilize or freeze dry the substances. Particularly, where it is necessary or beneficial to gently drying the substances, e.g. without heating them in an inappropriate manner, lyophilisation often is preferred.

For example, in many medical applications, pharmaceuticals or drug substances are to be, e.g., orally, intravenously, subcutaneously, or intrathecally administered in liquid form. As an example, for intravenous administration it is known to use infusion bags which can be hanged on a support and continuously drip a liquid drug substance or a drug diluent mixture through an infusion needle into a patient. However, in connection with liquid drug substances, many pharmaceuticals and particularly biopharmaceuticals cannot be stored and supplied for an appropriate duration in liquid form since they are commonly unstable in that form. More specifically, many antibiotics and biological drugs are unstable in liquid form such that their quality cannot be maintained as liquid. In particular, stress caused by shaking, microbiological growth, aggregation or the like may compromise the drugs. As mentioned, it is however known to supply the drug in a dry form, such as in a powder or the like, in which they are essentially more stable and robust compared to the liquid form. The dry drug formulation is then reconstituted or dissolved shortly before administration. Additionally, larger amounts of intermediate species of such drug formulations which are equally or less stabile can benefit from being dried prior to their further processing into drug formulations. By being dried the initial storage temperatures could be elevated from - 40°C or lower to ambient conditions or only 2° to 8°C refrigerated conditions.

To achieve or maintain appropriate hygienic and quality standards the substances are often carefully lyophilised under specific conditions such as an aseptic environment. Thereby, the substances are usually lyophilised in specific containers such as vials or bulk freeze drying trays from which they can be transferred to other containers or packages for storage and supply. Shortly before being applied or administered the substances are then reconstituted. Typically, such procedure is rather elaborate and prone to mistakes. When transferring the substances, there may be a risk of loss or contamination. For example, the lyophilized substance or lyophilisate is typically prone to breakage or become brittle upon exposure to mechanical stress such as transport or transfer of the lyophilisate. This can be of particular importance when the substances, such as highly potent drug substances, are provided at comparably small amounts and/or when precise amounts have to be handled. Additionally, this might endanger caregivers or clinicians when they need to prepare high potent drug substances by increasing the risk of exposure to the drug substance. Usually, comparably large efforts are made to protect the ones who need to prepare the drug substances for patients, for example by use of laminar air flow, isolator boxes, gloves over gloves and protective sleeves.

Furthermore, particularly on an industrial level, preparation of lyophilisates can be comparably inefficient. More specifically, commonly the often liquid substrate is provided in a container such as in a vial and the vial is then positioned in a freeze dryer. Often, freeze dryers are provided with shelves onto which the containers loaded with the substrate are positioned. Heat or thermal energy is provided to the lyophilisate via conduction by the shelves through the containers to the cakes, and via gas by convection and radiation from the walls and the doors. Such procedure, typically requires a comparably long time such as more than 24 hours or the like. Furthermore, it may be difficult to achieve a homogenous lyophilisate, as the heat transfer to the substrate inside the vial cannot exactly be predefined.

Still further, when pharmaceuticals or drug substances are involved, the persons handling the substances can be confronted with comparably high demands. Therefore, such applications are often prone to mistakes particularly when comparably low skilled or low educated persons are involved. For example, when administration in an infusion bag is aimed, the preparation of the drug substance including its reconstitution and addition in the infusion bag is of utmost importance to assure an appropriate treatment. Particularly, where highly potent drug substances are involved the treatment by infusion may be inappropriate if it cannot be assured that the preparation is performed appropriately, such as it is often the case in rather low and middle income countries. Also, in applications where the speed of preparation is crucial such as in emergency situations, the known preparation of highly potent drug substances often is not appropriately efficient. DE 10 2015 216725 A1 discloses a placing plate for a freeze drying facility.

Therefore, there is a need for a system or process allowing an efficient life cycle of a consumable lyophilisate starting from its preparation and ending at its administration.

### Disclosure of the Invention

According to the invention this need is settled by a procedure of drying a substrate as it is defined by the features of independent claim 1. Preferred embodiments are subject of the dependent claims.

In one aspect, a lyophilisate retainer for lyophilizing a substrate is disclosed. The retainer comprises a three dimensional scaffold body having pores. The pores of the scaffold body are configured to receive and hold the substrate. The scaffold body is made of a scaffold material providing heat transfer properties in lyophilisation to enable, facilitate or induce heat transfer to the substrate, particularly the entire substrate, held in the pores of the scaffold body.

Lyophilisation in the context of the present invention is a low temperature dehydration process, which involves freezing a substrate, lowering pressure and then removing ice by sublimation and desorption. The result from lyophilisation is the lyophilisate. Lyophilisation is also referred to as freeze drying. Lyophilisation can cover bulk freeze drying, which may produce lyophilized microspheres, or spray drying.

The substrate can be any substance intended to undergo lyophilisation. It can particularly be a liquid or fluid substance. The lyophilisate can be, e.g., a nutritional or dietary substance intended to be consumed by a person by drinking, eating or the like. It can also be an analytical substance or a substance to be used in a chemical process. However, preferably the lyophilisate is a lyophilised drug formulation, particularly, a highly potent drug formulation or intermediate thereof, which can comprise a biological compound such as a monoclonal antibody, an antibody drug conjugate, an antibody fragment, a locked nucleic acid (LNA), associated virus particles or the like.

The term "drug" as used herein relates to a therapeutically active agent, also commonly called active pharmaceutical ingredient (API), as well as to a combination of plural such therapeutically active substances. The term also encompasses diagnostic or imaging agents, like for example contrast agents (e.g. MRI contrast agents), tracers (e.g. PET tracers) and hormones, that need to be administered in liquid or high viscous form to the patient.

The term "drug formulation" as used herein relates to a single drug as defined above or a plurality of such drugs mixed or formulated. For example, besides the drug, a drug formulation may additionally comprise an excipient and/or other auxiliary ingredients. When being a dry drug formulation, the lyophilisate can be a solid drug formulation, a semisolid or powderous drug substance.

The term "drug substance" as used herein relates to a drug formulation as defined above in a form that is suitable for administration to the patient. Thereby, the drug substance can be the pure drug formulation or a drug formulation reconstituted, diluted or dissolved in an administrable form. A particularly preferred drug substance in the context of the invention is a solution, in particular a solution for oral or parenteral administration such as intravenous, subcutaneous, intrathecal or ophthalmic administration, being an injection or infusion.

The term "drug product" as used herein relates to a finished end product comprising a drug substance or a plurality of drug substances. In particular, a drug product may be a ready to use product having the drug substance in an appropriate dosage and/or in an appropriate form for administration. For example, a drug product may include a handling or storage device such as a flexible container.

The term "potency" used in connection with the drug formulation can be a measure of drug activity expressed in terms of the amount required to produce an effect of given intensity. Thus, the terms "high potency", "highly potent" or similar can relate to a formulation or substance which is active at comparably small amounts or dosages. In other words, a highly potent drug formulation can evoke a given response at comparably low concentration, while a drug formulation of lower potency can evoke the same response only at higher concentrations. The potency may depend on both the affinity and efficacy of the drug formulation. Handling such drug formulations or substances can be comparably challenging since comparably small variations in dosing can be detrimental.

In numbers, a highly potent drug formulation can be defined as a drug formulation having a biological activity at approximately 15 micrograms (µg) per kilogram (kg) of body weight or below in humans. This is equivalent to a therapeutic dose at approximately 1 milligrams (mg) or below in humans. The highly potent drug formulation can thus be defined as a drug having an inhalative Acceptable Daily Exposure (ADE) value of 1.5 µg/d or less, translating into an Indicative Occupational Exposure Limit (IOEL) value of 0.15 µg/m³. In particular, the highly potent drug formulation can be a class 3B drug or the like. When used with highly potent drug formulations to be administered by infusion, the invention can be particularly beneficial.

The term "three dimensional" in connection with the scaffold body can relate to extensions in all three directions in space. Thereby, compared to two dimensional bodies, such as sheet-like or plate-like bodies, which do not essentially extend in one direction in space, three dimensional bodies do have a spatial dimension.

The term "receive and hold the substrate" in connection with the pores can relate to a structure of holes or cavities dimensioned to be suitable for the substrate being provided into and staying inside the pores during processing of the lyophilisate retainer. Thereby, provision of the substrate into the pores can be achieved by immersing the scaffold body into the substrate or dispensing the substrate into the scaffold body. Holding the substrate in the pores can involve a retention due to a surface tension of the substrate or the like. Thereby, the higher the surface tension of the substrate is, the larger the diameter of the pores can be for still being capable of holding the substrate.

The pores of the scaffold body can be through holes or passages in the scaffold body. They can form a network of ducts in the scaffold body which is accessible through openings at a circumference or boundary of the scaffold body.

The pores can be configured to receive and hold the substrate by being properly shaped and dimensioned. Thereby, e.g., the diameter of the pores may depend on the type of substrate involved and the diameter of the pores can change within the scaffold body from the inside to the outside. For example, if the substrate has a comparably small viscosity and/or a comparably small surface tension, the pores can be configured to have comparably small diameters or the diameter of the pores can decrease from the inside of the scaffold body to the outside of the scaffold body to retain the less viscous material.

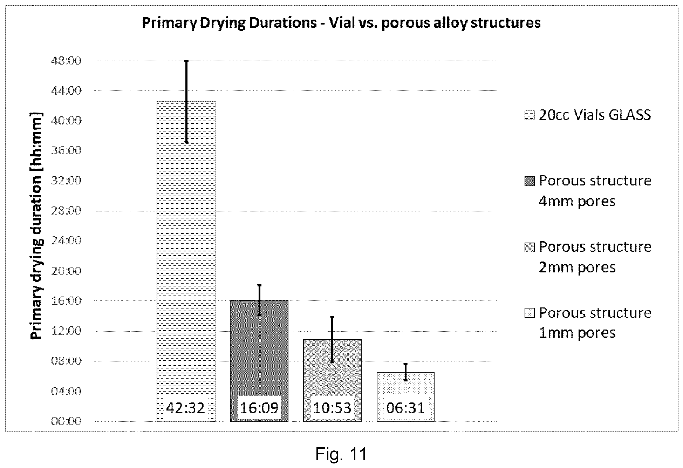

The lyophilisate retainer allows for an efficient heat transfer to the entire substrate during lyophilisation. In particular, due to the comparably large contact surface between the scaffold material and substrate when the substrate is received by the scaffold body, heat can efficiently be transferred to the substrate via the scaffold material. For example, when being arranged on a shelf of a freeze dryer, heat can be transferred from the shelf to the whole substrate distributed within the scaffold body via the scaffold material. Due to this efficient heat transfer, a considerably faster drying of the substrate is possible compared to common freeze drying in containers such as vials. For example, as shown in Fig. 11 experiments have shown that lyophilizing the same dosage of a specific substrate, which requires more than 42 hours in a conventional 20 mm diameter glass vial, takes around 16 hours in a lyophilisate retainer having an alloy scaffold body with pores of about 4 mm diameter, less than 11 hours in a lyophilisate retainer having an alloy scaffold body with pores of about 2 mm diameter and less than 7 hours in a lyophilisate retainer having an alloy scaffold body with pores of about 1 mm diameter. Thus, it could be shown that the increase in contact surface area between the scaffold body and the substrate is correlating with shortened primary drying times. By using the lyophilisate retainer, the efficiency of lyophilization process can be enhanced and, thus, the occupancy rate of the freeze dryer can be increased as well.

Furthermore, by using the lyophilisate retainer, the maximum distance between the heat transfer material, i.e. the scaffold material, and the substrate can be essentially decreased. This allows for achieving a comparably homogeneous heat distribution within the substrate during lyophilisation such that a comparably homogeneous drying of the lyophilisate is achieved.

Still further, the lyophilisate retainer allows for stabilizing the integrity of the lyophilisate. In particular, the scaffold material can be comparably robust such that the lyophilisate inside the pores of the scaffold body can be protected. Like this, the lyophilisate and particularly an accurate dosage thereof can efficiently be handled. Crumbling or breaking of the lyophilisate can be prevented by the scaffold body such that loss of lyophilisate can also be prevented. For example, transferring the lyophilisate held in the scaffold body into a diluent can conveniently and safely be performed. Like this, dosage accuracy of the lyophilisate and operational safety of the lyophilisate can be increased. Still, when being provided with a diluent, the lyophilisate can efficiently be dissolved or washed out of the scaffold body. Thus, preparation of the final product such as a drug product can be simple, accurate and efficient.

For being suitable to be used for lyophilisation of pharmaceutical or medical substances, the scaffold material advantageously is inert or can be efficiently be provided in an inert state. In any case, the scaffold material advantageously provides no or comparably limited chemical or other reaction with the pharmaceutical or medical substance to be lyophilized. Also, the scaffold material can be non-leaching. Thereby, it can particularly be configured to be sufficiently stable such that any passing of particles into the pharmaceutical or medical substance to be lyophilized is prevented.

Further, for achieving an efficient lyophilisation the scaffold material has to be configured to allow holding or keeping the substrate in the pores of the scaffold body. As substrates to be lyophilized typically are liquids, the scaffold material should not be hydrophobic or comprise hydrophobic portions where a contact with the substrate is intended. Rather, the scaffold material preferably is hydrophilic or uniformly hydrophilic particularly with regard to the substrate. More specifically, the scaffold material advantageously is uniformly hydrophilic at a surface of the scaffold body, where a contact with the substrate is intended such as in the pores of the scaffold body. Thus, the scaffold material can be configured such that a surface of the scaffold body forming the pores is hydrophilic.

Preferably, the scaffold material has a high thermal conductivity. Advantageous materials can be Aluminum such as an anodized Aluminum, an Aluminum alloy, stainless steel such as marine grade stainless steel, e.g. Society of Automative Engineers (SAE) 316L grade stainless steel, titanium or a polymeric material such as a biocompatible photopolymer, a polyamide or, more specifically, a olycaprolactam, a polylaurolactam or a poly(undecano-11-lactam) (polymers of Nylon). Thereby, the term "high thermal conductivity" can relate to a property of the scaffold body allowing an efficient heat transfer to the substrate, not only limited to the material but also to design properties that facilitate better heat distribution to the entire substrate. In particular, the thermal conductivity of the scaffold material preferably is 5 W/mK or more, or 10 W/mK or more, or 100 W/mK or more, or 200 W/mK or more, or 300 W/mK or more. Such a scaffold body allows for a particularly efficient heat transfer in a lyophilisation procedure.

Preferably, the pores of the scaffold body are configured to receive and hold the substrate by dimensioning a diameter of the pores in accordance with a surface tension and a viscosity of the substrate. In particular, the diameter of the pores can be shaped such that the surface tension of the substrate is sufficient for holding the substrate inside the pores.

Preferably, the scaffold body has end sides and each of the pores of the scaffold has an opening at one of the end sides. By providing the pores with an opening, they can efficiently be filled with the substrate.

Thereby, the pores of the scaffold body preferably are passageways extending between at least two of the end sides of the scaffold body. The involved end sides can particularly be opposite end sides of the scaffold body. Like this, the substrate can efficiently be provided into and dissolved out of the pores and, thus, the scaffold body.

Preferably, the pores of the scaffold body have a diameter in a range of about 0.5 mm to about 5 mm, particularly of about 1 mm to about 5 mm, of about 0.8 mm to about 1.2 mm, of about 1.8 mm to about 2.2 mm, or of about 3.5 mm to about 4.5 mm. Pores being dimensioned with such diameters are suitable for receiving and holding many substrates and particularly drug substrates.

Preferably, the scaffold body has a cylindrical or cubic shape. The cylindrical shape can be established by a cylinder having a circular, oval or polygonal base or cross section. Thereby, the base of the scaffold body preferably has a diameter of in between about 15 mm to about 60 mm. Such cylinders allow for an efficient handling of the lyophilisate retainer. In particular, the cylinders can be shaped similarly as vials such that handling equipment of vials can be used for handling the lyophilisate retainer. For long-term storage of larger quantities of intermediate substrate, the cylindric scaffold body can have a rectangular base or cross section or can be cubic. Thereby, the base of the scaffold may have a length of in between about 300 mm to about 800mm and a width of in between about 200 mm to about 500 mm. Such rectangles allow for an efficient handling of the intermediate substrate and are similarly shaped to the rectangular trays used for handling vials and bulk lyophilization trays in lab or large-scale lyophilizers.

Preferably, the scaffold body is a sponge-like structure. Such sponge-like structure allows for providing a comparably high ratio of pores relative to the scaffold material.

Preferably, the scaffold body has a frame structure made of the scaffold material. The frame structure may establish the scaffold body and provide specific properties for achieving an efficient loyphilisation.

In another aspect, a method of manufacturing a lyophilisate retainer for lyophilizing a substrate is disclosed. The method comprises a step of equipping the lyophilisate retainer with a three dimensional scaffold body made of a scaffold material having heat transfer properties to enable, induce or facilitate heat transfer to the preferably entire substrate during a lyophilisation process. Thereby, equipping the lyophilisate retainer with the scaffold body includes providing the scaffold body with pores configured to receive and hold the substrate.

The manufacturing method and its preferred examples described below allow for efficiently manufacturing the lyophilisate retainer and any of its preferred embodiments described above. Thereby, the effects and benefits described above in connection with the lyophilisate retainer and its preferred embodiments can be achieved as well.

For designing the pores of the scaffold body in an advantageous manner, properties of the substrate can be involved. Thereby, the method can include a step of analyzing the substrate or otherwise obtaining relevant properties of the substrate such as its viscosity or other properties.

When having the properties at hand the shape and dimension of the pores can be defined to allow a specifically efficient immersion and retaining of the substrate inside the pores. In particular, providing the scaffold body with pores preferably comprises dimensioning the pores of the scaffold body in accordance with a surface tension of the substrate. Additionally or alternatively, providing the scaffold body with pores preferably comprises dimensioning the pores of the scaffold body in accordance with a viscosity of the substrate. Additionally or alternatively, providing the scaffold body with a treated smooth or rough surface in accordance to the cohesion of the substrate.

Preferably, equipping the lyophilisate retainer with the scaffold body comprises additive manufacturing the scaffold body. The additive manufacturing can be a process of extruding the scaffold material, of binder dispensing the scaffold material, of laser sintering the scaffold material, of binder spraying the scaffold material or the like.

Advantageously, the additive manufacturing is a 3D printing process. Such additive manufacturing allows for efficiently generating comparably complex shapes of the scaffold body. Like this, a shape particularly suitable for the specific substrate can efficiently be created and embodied in the scaffold body.

Alternatively or additionally, providing the scaffold body with pores can include a subtractive manufacturing step which preferably comprises drilling the pores into the scaffold material. Such drilling can be an efficient alternative to the additive manufacturing of the scaffold body. Furthermore, an additive manufactured scaffold body can be complemented with such drilling.

Thereby, the drilling of the scaffold body can be performed by any suitable means such as mechanical drilling, laser drilling, water jet drilling or the like.

Preferably, the method comprising a step of treating a surface of the scaffold body to adapt its surface properties. For example, such treatment may include passivating the surface, anodizing the surface, or the like. By means of such treatment, loading of the substrate into the scaffold can be improved. Further, cohesion of the substrate to the pores or dilution of the substrate can be improved by adapting the surface properties by an appropriate treatment. Preferably, the surface properties comprise hydrophilicity and/or inertness.

The invention is a procedure of drying a substrate, comprising the steps of (i) obtaining a lyophilisate retainer as described above; (ii) soaking a scaffold body of the lyophilisate retainer with the substrate; and (iii) lyophilizing the substrate inside pores of the scaffold body of the lyophilisate retainer.

The procedure according to the invention allows for efficiently generating a lyophilisate which is provided in a robust and protected form. Thereby, the effects and benefits described above in connection with the lyophilisate retainer and its preferred embodiments can be achieved by the procedure according to the invention and its preferred embodiments described below.

Preferably, lyophilizing the substrate inside the pores of the scaffold body of the lyophilisate retainer comprises placing the lyophilisate retainer into a freeze dryer when the scaffold body of the lyophilisate retainer is soaked with the substrate. Like this an accelerated and more efficient lyophilisation process using a common freeze dryer can be achieved.

### Brief Description of the Drawings

The lyophilisate retainer, the method of manufacturing a lyophilisate retainer and the procedure of drying a substrate according to the invention are described in more detail herein below by way of exemplary embodiments and with reference to the attached drawings, in which:
- Fig. 1: shows a first example of a method for manufacturing a first example of a lyophilisate retainer;
- Fig. 2: shows a second example of a method for manufacturing a second example of a lyophilisate retainer;
- Fig. 3: shows a top view on the lyophilisate retainer of Fig. 2;
- Fig. 4: shows a cross sectional view of the lyophilisate retainer of Fig. 2 along the line A-A of Fig. 3;
- Fig. 5: shows a perspective view of a third embodiment of a lyophilisate retainer according to the invention;
- Fig. 6: shows a top view on the lyophilisate retainer of Fig. 5;
- Fig. 7: shows a cross sectional view of the lyophilisate retainer of Fig. 5 along the line B-B of Fig. 6;
- Fig. 8: shows a perspective view of one of the segments of which the lyophilisate retainer of Fig. 5 is composed of;
- Fig. 9: shows an embodiment of a procedure of drying a substrate according to the invention involving the first example of the lyophilisate retainer;
- Fig. 10: shows the lyophilisate retainer of Fig. 2 after being involved in the procedure of Fig. 9; and
- Fig. 11: shows a chart of results of experimentally gathered drying times of lyophilisate retainers compared to conventional glass vial drying time.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

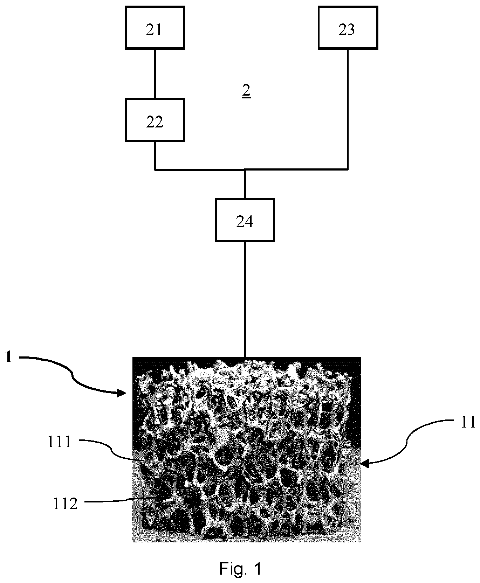

Fig. 1 shows a first example of a method 2 of manufacturing a lyophilisate retainer for lyophilizing a substrate. The first manufacturing method 2 comprises a step 21 of obtaining properties of a fluid drug substance as substrate. For example, properties like the viscosity, density and the surface tension of the drug substance can be measured or evaluated. Then, in step 22 a scaffold body of the lyophilisate retainer is designed. Thereby, dimension and shape of pores of the scaffold body are defined in accordance with the properties of the drug substance. For example, an average diameter or a range of diameters of the pores is calculated by considering the surface tension of the drug substance.

In parallel, in a step 23 an aluminum alloy powder such as a powder of AlSi₁₀Mg or the like is obtained. Thereby, an alloy is chosen which allows to provide for a robust construction, a good compatibility to the substrate, inertness to the substrate, an appropriate hydrophilicity for the substrate and a good heat transfer. For example, the alloy has a thermal conductivity of 10 W/mK or more. In step 24 the powder is filled into a 3D printer and data on the design of the scaffold body is loaded into the 3D printer. The 3D printer then prints the scaffold body 11 of a first example of a lyophilisate retainer 1.

The lyophilisate retainer 1 generally is sponge-like shaped. It has a frame structure 111 and pores 112. As mentioned above, the pores are shaped and dimensioned to receive and hold the drug substance. The scaffold body 11 is cylindrical with upper and lower end sides as well as a circumferential lateral end side. Each of the pores 112 of the scaffold has an opening at one of the end sides. More specifically, the pores form a network of passageways in the scaffold body 11 extending between at least two of the end sides.

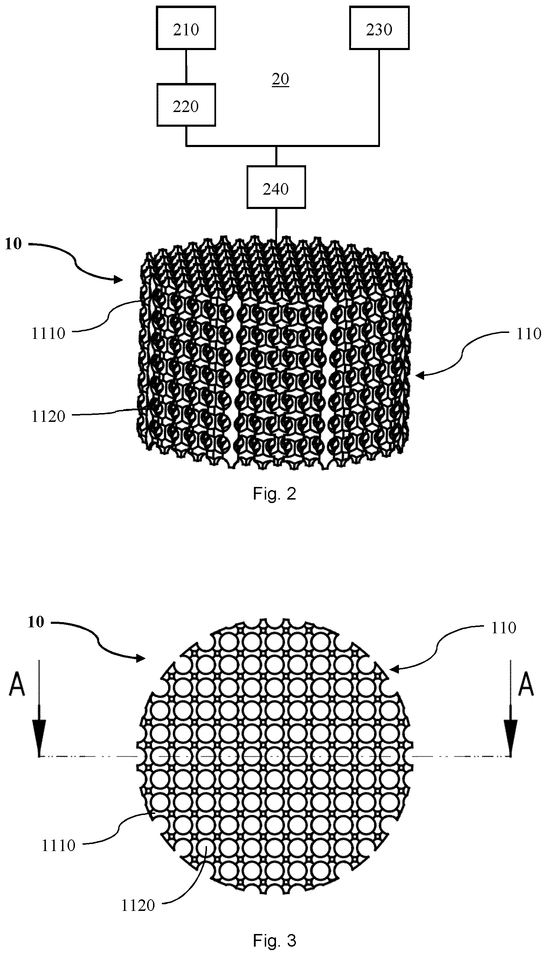

In Fig. 2 a second example of a method 20 of manufacturing a lyophilisate retainer 10 for lyophilizing a substrate is shown. The second manufacturing method 20 comprises a step 210 of obtaining properties of a fluid drug substance as substrate. Again, properties like the viscosity, density and the surface tension of the drug substance can be measured or evaluated. Then, in step 220 a scaffold body of the lyophilisate retainer is designed. Thereby, dimension and shape of the pores is defined in accordance with the properties of the drug substance.

In a step 230 a block of an aluminum alloy is obtained and formed to a cylinder. In step 240, the pores in accordance with the design of the scaffold body are provided into the cylindrical block. More specifically, through bores are drilled through the cylindrical block by means of a mechanical drill, such that a scaffold body 110 of the second example of a lyophilisate retainer 10 results.

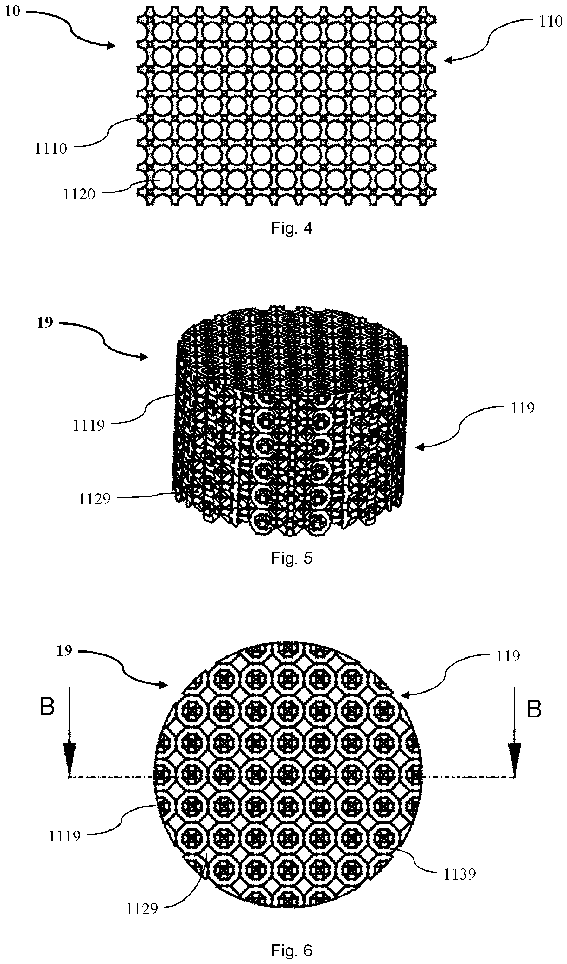

The scaffold body 110 generally is cylindrical, wherein parallel vertical and horizontal bores 1120 are drilled through it. As can be best seen in the top view of Fig. 3, the vertical bores 1120 are straight and extend form an upper to a lower end side of the scaffold body 110. Furthermore, as can be best seen in the cross-sectional view of Fig. 4 the horizontal bores 1120 are straight and extend from one lateral end side to an opposite one. From Fig. 4 it can also be derived that to the first set of horizontal bores another set of horizontal bores is extending orthogonally to the first set from one lateral end side to an opposite one. Thereby, the vertical and horizontal bores 1120 are interconnected such that a network of passageways is established. The portion of the cylinder block not being removed by drilling establishes a frame structure 1110 of the scaffold body 110. Thereby, the bores 1120 form pores being shaped and dimensioned to receive and hold the drug substance.

Fig. 5 shows a third example of a lyophilisate retainer 19. The lyophilisate retainer is manufactured by a method similar to the first method 2 described above. More specifically, properties of a fluid drug substance as substrate are obtained and a scaffold body of the lyophilisate retainer 19 is designed. Then, a Nylon base substance is obtained and filled into a 3D printer. Further, data about the design of the scaffold body is loaded to the 3D printer. The 3D printer then prints a scaffold body 119 of the lyophilisate retainer 19.

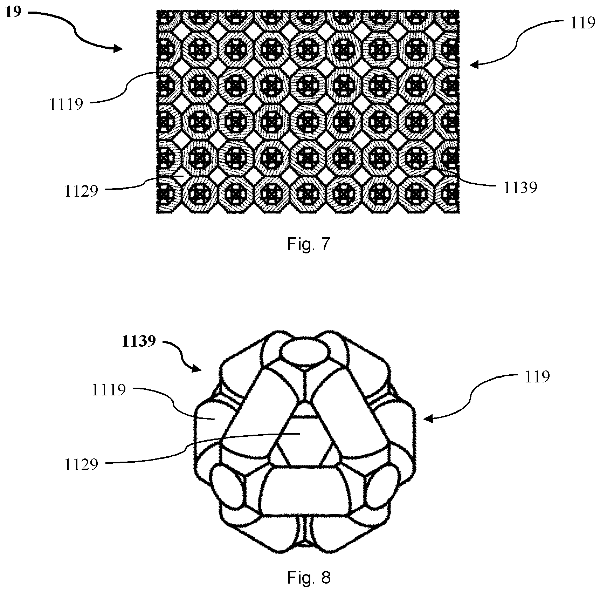

More specifically, the scaffold body 119 is composed of a plurality of three dimensional elements 1139 as shown in Fig. 8. As can be seen in the top view of Fig. 6 and the cross sectional view of Fig. 7 the structure formed by the elements 1139 form pores 1129 which comprise horizontal and vertical passages extending top-down and laterally through the scaffold body 119. By providing a specific network of passageways, the scaffold body 119 is embodied to receive and hold the specific substrate or drug substance it is designed for.

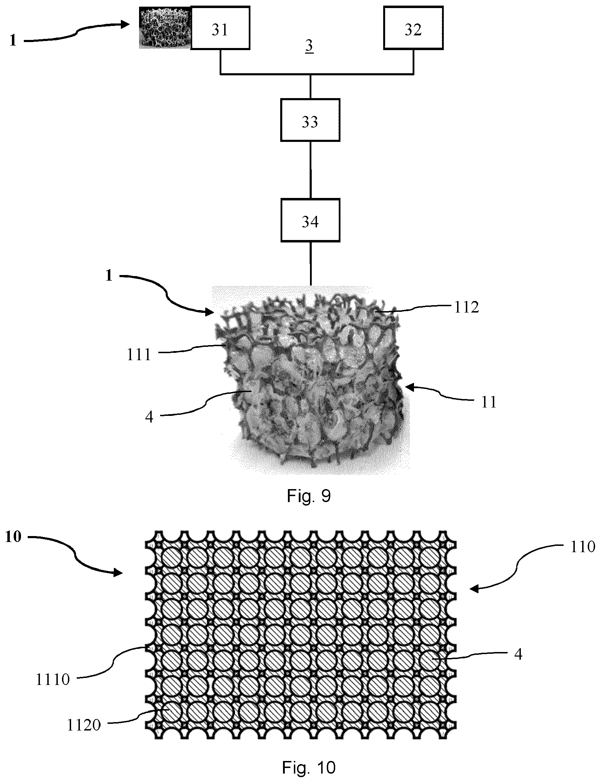

In Fig. 9 an embodiment of a procedure 3 of drying a fluid drug substance as substrate according to the invention is shown. The procedure 3 comprises a step 31 of obtaining the first lyophilisate retainer 1 and a step 32 of preparing and obtaining the drug substance in a containment. In a step 33, the lyophilisate retainer 1 is soaked with the drug substance by placing the scaffold body 11 into the containment for a certain time or by dispensing the substrate or drug substance into the lyophilisate retainer 1. By lowering the temperature below the freezing point of the substrate or drug substance the entrapment can be ensured. Thereby, the drug substance is trapped in the pores 112 of the scaffold body 11.

In a step 34, the lyophilisate retainer 1 is transferred from the containment onto a shelf of a freeze dryer. Thereby, the drug substance is held in the pores 112 of the scaffold body 11. There, the drug substance is lyophilised wherein heat is transferred from the shelf to the drug substance inside the pores 112 via the frame structure 111. After being processed in the freeze dryer, the lyophilisate 4 of the drug substance is embedded inside the pores 112 of the scaffold body 11. Thereby, the frame structure 111 of the scaffold body 11 provides stability and protection to the lyophilisate 4.

Even though Fig. 9 shows the procedure 3 involving the lyophilisate retainer 1 of Fig. 1, the procedure 3 can likewise be applied with the lyophilisate retainer 10 of Fig. 2 or with the lyophilisate retainer 19 of Fig. 5. Thus, Fig. 9 can alternatively be depicted with the lyophilisate retainer 10 or the lyophilisate retainer 19. For example, Fig. 10 shows the lyophilisate retainer 10 being processed by the procedure 3 such that the lyophilisate 4 of the drug substance is arranged inside the pores 1120 of the scaffold body 110.

This description and the accompanying drawings that illustrate aspects and embodiments of the present invention should not be taken as limiting the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the scope of the claims. In some instances, well-known circuits, structures and techniques have not been shown in detail in order not to obscure the invention. Thus, it will be understood that changes and modifications may be made by those of ordinary skill within the scope of the following claims. In particular, the present invention covers further embodiments with any combination of features from different embodiments described above and below.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. A procedure (3) of drying a substrate, comprising:
obtaining a lyophilisate retainer (1; 10; 19) for lyophilizing a substrate **characterized in that** the lyophilisate retainer (1; 10; 19) comprises:
a three dimensional scaffold body (11; 110; 119) having pores (112; 1120; 1129), wherein
the pores (112; 1120; 1129) of the scaffold body (11; 110; 119) are configured to receive and hold the substrate, and
the scaffold body (11; 110; 119) is made of a scaffold material providing heat transfer properties in lyophilisation to enable heat transfer to the substrate held in the pores (112; 1120; 1129) of the scaffold body (11; 110; 119);
soaking the scaffold body (11; 110; 119) of the lyophilisate retainer (1; 10; 19) with the substrate; and
lyophilizing the substrate inside the pores (112; 1120; 1129) of the scaffold body (11; 110; 119) of the lyophilisate retainer (1; 10; 19).

2. The procedure (3) of claim 1, wherein lyophilizing the substrate inside the pores (112; 1120; 1129) of the scaffold body (11; 110; 119) of the lyophilisate retainer (1; 10; 19) comprises placing the lyophilisate retainer (1; 10; 19) into a freeze dryer when the scaffold body (11; 110; 119) of the lyophilisate retainer (1; 10; 19) is soaked with the substrate.

3. The procedure (3) of claim 1 or 2, wherein the scaffold material of the lyophilisate retainer (1; 10; 19) has a high thermal conductivity.

4. The procedure (3) of claim 1, wherein the thermal conductivity of the scaffold material of the lyophilisate retainer (1; 10; 19) is 5 W/mK or more.

5. The procedure (3) of any one of the preceding claims, wherein the scaffold material of the lyophilisate retainer (1; 10; 19) is inert.

6. The procedure (3) of any one of the preceding claims, wherein the scaffold material of the lyophilisate retainer (1; 10; 19) is hydrophilic and particularly uniformly hydrophilic.

7. The procedure (3) of any one of the preceding claims, wherein the pores (112; 1120; 1129) of the scaffold body (11; 110; 119) of the lyophilisate retainer (1; 10; 19) are configured to receive and hold the substrate by dimensioning a diameter of the pores (112; 1120; 1129) in accordance with a surface tension and a viscosity of the substrate.

8. The procedure (3) of any one of the preceding claims, wherein the scaffold body (11; 110; 119) of the lyophilisate retainer (1; 10; 19) has end sides and each of the pores (112; 1120; 1129) of the scaffold body (11; 110; 119) has an opening at one of the end sides.

9. The procedure (3) of claim 8, wherein the pores (112; 1120; 1129) of the scaffold body (11; 110; 119) of the lyophilisate retainer (1; 10; 19) are passageways extending between at least two of the end sides of the scaffold body (11; 110; 119) of the lyophilisate retainer (1; 10; 19).

10. The procedure (3) of any one of the preceding claims, wherein the pores (112; 1120; 1129) of the scaffold body (11; 110; 119) of the lyophilisate retainer (1; 10; 19) have a diameter in a range of about 0.5 mm to about 5 mm, of about 1 mm to about 5 mm, of about 0.8 mm to about 1.2 mm, of about 1.8 mm to about 2.2 mm, or of about 3.5 mm to about 4.5 mm.

11. The procedure (3) of any one of the preceding claims, wherein the scaffold body (11; 110; 119) of the lyophilisate retainer (1; 10; 19) has a cylindrical shape.

12. The procedure (3) of claim 11, wherein a base of the scaffold body (11; 110; 119) of the lyophilisate retainer (1; 10; 19) has a diameter of in between about 15 mm to about 60 mm.

13. The procedure (3) of any one of the preceding claims, wherein the scaffold body (11; 110; 119) of the lyophilisate retainer (1; 10; 19) is a sponge-like structure.

14. The procedure (3) of any one of the preceding claims, wherein the scaffold body (11; 110; 119) of the lyophilisate retainer (1; 10; 19) has a frame structure (111; 1110; 1119) made of the scaffold material.

## Patentansprüche

1. Verfahren (3) zum Trocknen eines Substrats, umfassend:
Erhalten eines Lyophilisathalters (1; 10; 19) zum Lyophilisieren eines Substrats, **dadurch gekennzeichnet, dass** der Lyophilisathalter (1; 10; 19) umfasst:
einen dreidimensionalen Gerüstkörper (11; 110; 119) mit Poren (112; 1120; 1129), wobei
die Poren (112; 1120; 1129) des Gerüstkörpers (11; 110; 119) zum Aufnehmen und Halten des Substrats ausgebildet sind und
der Gerüstkörper (11; 110; 119) aus einem Gerüstmaterial gefertigt ist, das bei der Lyophilisierung Wärmeübertragungseigenschaften bereitstellt, um eine Wärmeübertragung auf das in den Poren (112; 1120; 1129) des Gerüstkörpers (11; 110; 119) gehaltene Substrat zu ermöglichen;
Tränken des Gerüstkörpers (11; 110; 119) des Lyophilisathalters (1; 10; 19) mit dem Substrat und
Lyophilisieren des Substrats in den Poren (112; 1120; 1129) des Gerüstkörpers (11; 110; 119) des Lyophilisathalters (1; 10; 19).

2. Verfahren (3) nach Anspruch 1, wobei das Lyophilisieren des Substrats in den Poren (112; 1120; 1129) des Gerüstkörpers (11; 110; 119) des Lyophilisathalters (1; 10; 19) das Platzieren des Lyophilisathalters (1; 10; 19) in einen Gefriertrockner umfasst, wenn der Gerüstkörper (11; 110; 119) des Lyophilisathalters (1; 10; 19) mit dem Substrat getränkt ist.

3. Verfahren (3) nach Anspruch 1 oder 2, wobei das Gerüstmaterial des Lyophilisathalters (1; 10; 19) eine hohe Wärmeleitfähigkeit aufweist.

4. Verfahren (3) nach Anspruch 1, wobei die Wärmeleitfähigkeit des Gerüstmaterials des Lyophilisathalters (1; 10; 19) 5 W/mK oder mehr beträgt.

5. Verfahren (3) nach einem der vorstehenden Ansprüche, wobei das Gerüstmaterial des Lyophilisathalters (1; 10; 19) inert ist.

6. Verfahren (3) nach einem der vorstehenden Ansprüche, wobei das Gerüstmaterial des Lyophilisathalters (1; 10; 19) hydrophil und insbesondere einheitlich hydrophil ist.

7. Verfahren (3) nach einem der vorstehenden Ansprüche, wobei die Poren (112; 1120; 1129) des Gerüstkörpers (11; 110; 119) des Lyophilisathalters (1; 10; 19) zum Aufnehmen und Halten des Substrats ausgebildet sind, indem ein Durchmesser der Poren (112; 1120; 1129) gemäß einer Oberflächenspannung und einer Viskosität des Substrats bemessen ist.

8. Verfahren (3) nach einem der vorstehenden Ansprüche, wobei der Gerüstkörper (11; 110; 119) des Lyophilisathalters (1; 10; 19) Endseiten aufweist und jede der Poren (112; 1120; 1129) des Gerüstkörpers (11; 110; 119) eine Öffnung auf einer der Endseiten aufweist.

9. Verfahren (3) nach Anspruch 8, wobei die Poren (112; 1120; 1129) des Gerüstkörpers (11; 110; 119) des Lyophilisathalters (1; 10; 19) Durchgänge sind, die sich zwischen mindestens zwei der Endseiten des Gerüstkörpers (11; 110; 119) des Lyophilisathalters (1; 10; 19) erstrecken.

10. Verfahren (3) nach einem der vorstehenden Ansprüche, wobei die Poren (112; 1120; 1129) des Gerüstkörpers (11; 110; 119) des Lyophilisathalters (1; 10; 19) einen Durchmesser im Bereich von etwa 0,5 mm bis etwa 5 mm, von etwa 1 mm bis etwa 5 mm, von etwa 0,8 mm bis etwa 1,2 mm, von etwa 1,8 mm bis etwa 2,2 mm oder von etwa 3,5 mm bis etwa 4,5 mm aufweisen.

11. Verfahren (3) nach einem der vorstehenden Ansprüche, wobei der Gerüstkörper (11; 110; 119) des Lyophilisathalters (1; 10; 19) eine zylindrische Form aufweist.

12. Verfahren (3) nach Anspruch 11, wobei eine Basis des Gerüstkörpers (11; 110; 119) des Lyophilisathalters (1; 10; 19) einen Durchmesser zwischen etwa 15 mm und etwa 60 mm aufweist.

13. Verfahren (3) nach einem der vorstehenden Ansprüche, wobei der Gerüstkörper (11; 110; 119) des Lyophilisathalters (1; 10; 19) eine schwammartige Struktur ist.

14. Verfahren (3) nach einem der vorstehenden Ansprüche, wobei der Gerüstkörper (11; 110; 119) des Lyophilisathalters (1; 10; 19) eine Rahmenstruktur (111; 1110; 1119) aufweist, die aus dem Gerüstmaterial gefertigt ist.

## Revendications

1. Procédé (3) de séchage d'un substrat, comprenant :
l'obtention d'un dispositif de retenue de lyophilisat (1 ; 10 ; 19) pour lyophiliser un substrat **caractérisé en ce que** le dispositif de retenue de lyophilisat (1 ; 10 ; 19) comprend :
un corps d'échafaudage tridimensionnel (11 ; 110 ; 119) ayant des pores (112 ; 1120 ; 1129), dans lequel
les pores (112 ; 1120 ; 1129) du corps d'échafaudage (11 ; 110 ; 119) sont conçus pour recevoir et maintenir le substrat, et
le corps d'échafaudage (11 ; 110 ; 119) est constitué d'un matériau d'échafaudage fournissant des propriétés de transfert de chaleur lors de la lyophilisation pour permettre un transfert de chaleur au substrat maintenu dans les pores (112 ; 1120 ; 1129) du corps d'échafaudage (11 ; 110 ; 119) ;
l'imbibition du corps d'échafaudage (11 ; 110 ; 119) du dispositif de retenue de lyophilisat (1 ; 10 ; 19) avec le substrat ; et
la lyophilisation du substrat à l'intérieur des pores (112 ; 1120 ; 1129) du corps d'échafaudage (11 ; 110 ; 119) du dispositif de retenue de lyophilisat (1 ; 10 ; 19).

2. Procédé (3) selon la revendication 1, dans lequel la lyophilisation du substrat à l'intérieur des pores (112 ; 1120 ; 1129) du corps d'échafaudage (11 ; 110 ; 119) du dispositif de retenue de lyophilisat (1 ; 10 ; 19) comprend le placement du dispositif de retenue de lyophilisat (1 ; 10 ; 19) dans un lyophilisateur lorsque le corps d'échafaudage (11 ; 110 ; 119) du dispositif de retenue de lyophilisat (1 ; 10 ; 19) est imbibé avec le substrat.

3. Procédé (3) selon la revendication 1 ou 2, dans lequel le matériau d'échafaudage du dispositif de retenue de lyophilisat (1 ; 10 ; 19) a une conductivité thermique élevée.

4. Procédé (3) selon la revendication 1, dans lequel 1a conductivité thermique du matériau d'échafaudage du dispositif de retenue de lyophilisat (1 ; 10 ; 19) est de 5 W/mK ou plus.

5. Procédé (3) selon l'une quelconque des revendications précédentes, dans lequel le matériau d'échafaudage du dispositif de retenue de lyophilisat (1 ; 10 ; 19) est inerte.

6. Procédé (3) selon l'une quelconque des revendications précédentes, dans lequel le matériau d'échafaudage du dispositif de retenue de lyophilisat (1 ; 10 ; 19) est hydrophile et en particulier uniformément hydrophile.

7. Procédé (3) selon l'une quelconque des revendications précédentes, dans lequel les pores (112 ; 1120 ; 1129) du corps d'échafaudage (11 ; 110 ; 119) du dispositif de retenue de lyophilisat (1 ; 10 ; 19) sont conçus pour recevoir et maintenir le substrat en dimensionnant un diamètre des pores (112 ; 1120 ; 1129) conformément à une tension de surface et une viscosité du substrat.

8. Procédé (3) selon l'une quelconque des revendications précédentes, dans lequel le corps d'échafaudage (11 ; 110 ; 119) du dispositif de retenue de lyophilisat (1 ; 10 ; 19) a des côtés d'extrémité et chacun des pores (112 ; 1120 ; 1129) du corps d'échafaudage (11 ; 110 ; 119) a une ouverture au niveau de l'un des côtés d'extrémité.

9. Procédé (3) selon la revendication 8, dans lequel les pores (112 ; 1120 ; 1129) du corps d'échafaudage (11 ; 110 ; 119) du dispositif de retenue de lyophilisat (1 ; 10 ; 19) sont des passages s'étendant entre au moins deux des côtés d'extrémité du corps d'échafaudage (11 ; 110 ; 119) du dispositif de retenue de lyophilisat (1 ; 10 ; 19).

10. Procédé (3) selon l'une quelconque des revendications précédentes, dans lequel les pores (112 ; 1120 ; 1129) du corps d'échafaudage (11 ; 110 ; 119) du dispositif de retenue de lyophilisat (1 ; 10 ; 19) ont un diamètre dans une plage d'environ 0,5 mm à environ 5 mm, d'environ 1 mm à environ 5 mm, d'environ 0,8 mm à environ 1,2 mm, d'environ 1,8 mm à environ 2,2 mm, ou d'environ 3,5 mm à environ 4,5 mm.

11. Procédé (3) selon l'une quelconque des revendications précédentes, dans lequel le corps d'échafaudage (11 ; 110 ; 119) du dispositif de retenue de lyophilisat (1 ; 10 ; 19) a une forme cylindrique.

12. Procédé (3) selon la revendication 11, dans lequel une base du corps d'échafaudage (11 ; 110 ; 119) du dispositif de retenue de lyophilisat (1 ; 10 ; 19) a un diamètre compris entre environ 15 mm et environ 60 mm.

13. Procédé (3) selon l'une quelconque des revendications précédentes, dans lequel le corps d'échafaudage (11 ; 110 ; 119) du dispositif de retenue de lyophilisat (1 ; 10 ; 19) est une structure semblable à une éponge.

14. Procédé (3) selon l'une quelconque des revendications précédentes, dans lequel le corps d'échafaudage (11 ; 110 ; 119) du dispositif de retenue de lyophilisat (1 ; 10 ; 19) a une structure de cadre (111 ; 1110 ; 1119) constituée du matériau d'échafaudage.
